(19) Europäisches Patentamt
European Patent Office
Office européen des brevets

(11) Veröffentlichungsnummer: **0 419 965 A2**

# (12) EUROPÄISCHE PATENTANMELDUNG

(21) Anmeldenummer: **90117704.8**

(22) Anmeldetag: **14.09.90**

(51) Int. Cl.5: **C07D 251/34**, C08G 18/80

(30) Priorität: **27.09.89 DE 3932168**

(43) Veröffentlichungstag der Anmeldung:
**03.04.91 Patentblatt 91/14**

(84) Benannte Vertragsstaaten:
**AT BE CH DE ES FR GB IT LI NL SE**

(71) Anmelder: **BAYER AG**
**W-5090 Leverkusen 1 Bayerwerk(DE)**

(72) Erfinder: **Halpaap, Reinhard, Dr.**
**In der Hildscheid 6**
**W-5068 Odenthal-Glöbusch(DE)**
Erfinder: **Dünwald, Wilhelm, Dr.**
**Geschw.-Scholl-Strasse 16**
**W-5090 Leverkusen 1(DE)**
Erfinder: **Casselmann, Holger, Dr.**
**Töpferweg 36**
**W-5060 Bergisch Gladbach 2(DE)**
Erfinder: **Schlegel, Hans**
**Habichtgasse 4**
**W-5090 Leverkusen 1(DE)**

## (54) Lackpolyisocyanate, ein Verfahren zu ihrer Herstellung und ihre Verwendung.

(57) Lackpolyisocyanate, gekennzeichnet durch das gleichzeitige Vorliegen von 0,5 bis 15 Gew.-% an Triazineinheiten, von 5 bis 20 Gew.-% an Isocyanatgruppen in blockierter Form und 0 bis 30 Gew-% an sonstigen, von den blockierten Isocyanatgruppen verschiedenen, chemisch eingebauten Urethangruppen, ein Verfahren zur Herstellung dieser Lackpolyisocyanate durch Umsetzung von a) organischen Diisocyanaten mit b) Blockierungsmitteln für Isocyanatgruppen, c) Aminogruppen aufweisenden Triazinen und gegebenenfalls d) organischen Polyhydroxylverbindungen des Molekulargewichtsbereichs 62 bis 3.000 und die Verwendung der Lackpolyisocyanate in Kombination mit organischen Polyhydroxylverbindungen als Einbrennlack oder zur Herstellung eines Einbrennlacks.

EP 0 419 965 A2

# LACKPOLYISOCYANATE, EIN VERFAHREN ZU IHRER HERSTELLUNG UND IHRE VERWENDUNG

Die vorliegende Erfindung betrifft neue Lackpolyisocyanate mit blockierten Isocyanatgruppen, die u.a. durch das Vorliegen von über Harnstoffgruppen eingebauten 1,3,5-Triazin-Strukturelementen gekennzeichnet sind, ein Verfahren zur Herstellung dieser Lackpolyisocyanate sowie ihre Verwendung in Kombination mit organischen Hydroxylverbindungen als Einbrennlack oder zur Herstellung eines Einbrennlacks.

Die Verwendung von 1,3,5-Triazin-Derivaten, insbesondere von Melamin als Ausgangsmaterial zur Herstellung von Lackbindemitteln ist seit langem bekannt:

N-Methylolmelaminharze und entsprechende alkoxylierte Verbindungen sind als Vernetzer für Lacke bekannt; vergl. z.B.: T. Yamamoto, T. Nakamichi und O. Ohe in J. of Coat. Technology 60 , 51 (1988) oder H. Dürr und M Schön in Kunstharznachrichten 45 , 17 (1986).

Die Verwendung von Melamin als Komponente in Polyurethan-Harzen oder Polyurethan-Beschichtungsmassen ist ebenfalls bereits bekannt. So werden in der DE-OS 2 737 402 Isocyanatgruppen-haltige Additionsprodukte aus Melamin und aromatischen Diisocyanaten als einbaufähige Füllstoffe beschrieben

Demgegenüber werden in der EP-A-56 153 Epoxidgruppenaufweisende Verbindungen aus Melaminhaltigen Triisocyanaten und Glycid als in Polyurethan-Harze einbaubare Füllstoffe mit erniedrigten Schmelzpunkten und verbesserter Verträglichkeit beschrieben.

Zur Herstellung duroplastischer Beschichtungen für Glasflaschen mit besonders guter Haftung werden entsprechend der DE-OS 2 938 309 Isocyanatprepolymere mit freiem Melamin, das in Form einer Polyester/Melamin-Paste eingesetzt wird, umgesetzt.

In der DE-OS 3 609 687 werden duroplastische Harze aus z.B. Diphenylmethandiisocyanat und Melamin beschrieben. Unter Carbodiimidisierung des Isocyanats werden Polycarbodiimid/Melamin-Mischungen als Schaum oder Pulver hergestellt, die bei 230 °C zu hitze- und flammfesten Harzen aushärten.

Reaktionsprodukte aus Diisocyanaten und Melamin im Äquivalentverhältnis $NCO/NH_2$ = 1 : 1 werden z.B. entsprechend der DE-OS 2 844 132 als Komponente für flammhemmende Beschichtungsmassen eingesetzt oder entsprechend der Japanischen Anmeldung JP 59 041-320 (Derwent 84-096 976) als hitzebeständige Harze verwendet.

Ebenfalls bekannt sind blockierte Polyisocyanate. Sie finden in der Polyurethanchemie zur Herstellung von Polyurethanlacken breite Anwendung. Zusammenfassende Darstellungen über die Chemie blockierter Isocyanate gibt z.B Z.W. Wicks in Progress of Organic Coatings 3 , 73 - 99 (1975) und 9 , 3 - 28 (1981).

1,3,5-Triazingruppen-haltige Polyisocyanate sind hochschmelzende und in üblichen organischen Lösungsmitteln schwerlösliche Verbindungen, vergl. DE-OS 2 737 402, Seite 7, Zeilen 23 - 25, Seite 8, Zeilen 15 - 19; EP-A-56 153, Seite 1, Zeilen 12 - 15, Seite 7, Zeilen 24 - 27 und DE-OS 3 609 687, Seite 15, Zeilen 18 - 20.

Obwohl der Einbau von 1,3,5-Triazin-Strukturelementen Polyurethankunststoffen oftmals erwünschte vorteilhafte Eigenschaften verleiht, und obwohl, wie der vorgenannte Stand der Technik zeigt, es nicht an Versuchen gefehlt hat, die vorteilhaften Eigenschaften der 1,3,5-Triazin-Struktureinheiten in Polyurethanen auszunutzen, fanden 1,3,5-Triazingruppen enthaltende Polyisocyanate wegen ihres hohen Schmelzpunkts und ihrer schlechten Löslichkeit in organischen Lösungsmitteln bislang kaum praktische Verwendung. Insbesondere als Lackrohstoffe sind in gängigen physiologisch unbedenklichen Lösungsmitteln trübungsfrei lösliche Produkte gefragt.

Es war daher die der Erfindung zugrunde liegende Aufgabe, 1,3,5-Triazin-Strukturelemente aufweisende Polyisocyanate zur Verfügung zu stellen, die die genannten Nachteile nicht aufweisen, die niedrig schmelzend und in gängigen Lacklösemitteln löslich sind. Sie sollten einfach herstellbar und als Lösung oder lösliches Festharz mit gegenüber Isocyanatgruppen reaktionsfähigen Verbindungen, insbesondere Polyhydroxylverbindungen zu hochwertigen Polyurethan-Kunststoffen, insbesondere Lackschichten umsetzbar sein.

Diese Aufgabe konnte durch die Bereitstellung der nachstehend näher beschriebenen Lackpolyisocyanate mit blockierten Isocyanatgruppen gelöst werden. Unter "Lackpolyisocyanaten" sind im Rahmen der Erfindung sowohl Polyisocyanate, d.h. einzelne bestimmte Verbindungen als auch Gemische von Polyisocyanaten mit blockierten Isocyanatgruppen zu verstehen, wie sie bei der Durchführung des erfindungsgemäßen Verfahrens anfallen.

Der Erfindung lag die überraschende Beobachtung zugrunde, daß blockierte, 1,3,5-Triazin-Strukturelementeaufweisende Polyisocyanate im Unterschied zu den entsprechenden unblockierten Polyisocyanaten einen vergleichsweise niedrigen Schmelzpunkt und eine gute Löslichkeit in üblichen Lacklösungsmitteln aufweisen. Dieser grundsätzliche Unterschied zwischen den blockierten Isocyanaten einerseits und den unblockierten Polyisocyanaten andererseits war keineswegs naheliegend, da er bei den üblichen Polyisocyanaten der Polyurethan-Chemie im allgemeinen nicht zu beobachten ist.

Gegenstand der Erfindung sind Lackpolyisocyanate mit blockierten Isocyanatgruppen, gekennzeichnet durch das gleichzeitige Vorliegen von

(i) 0,5 - 15 Gew.-% an über Harnstoffgruppen eingebauten Struktureinheiten der Formel

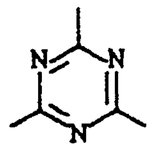

,

(ii) 5 - 20 Gew.-% an Isocyanatgruppen (berechnet als -NCO) in mit Blockierungsmitteln für Isocyanatgruppen blockierter Form und

(iii) 0 - 30 Gew.-% an sonstigen, von den blockierten Isocyanatgruppen verschiedenen, chemisch eingebauten Urethangruppen der Formel

-NH-CO-O- .

Gegenstand der Erfindung ist auch ein Verfahren zur Herstellung dieser Lackpolyisocyanate, welches dadurch gekennzeichnet ist, daß man

a) organische Diisocyanate des Molekulargewichtsbereichs 160 - 300 mit

b) Blockierungsmitteln für Isocyanatgruppen,

c) Aminogruppen aufweisenden Triazinen der Formel

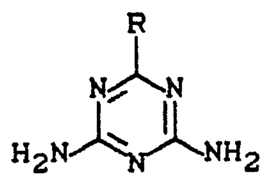

in welcher

R für eine Aminogruppe oder einen gegenüber Isocyanatgruppen inerten Substituenten steht

und gegebenenfalls

d) organischen Polyhydroxylverbindungen des Molekulargewichtsbereichs 62 - 3.000

umsetzt, wobei die Menge des Blockierungsmittels b) in einer zur Blockierung von 20 - 90 % der Isocyanatgruppen der Komponente a) ausreichenden Menge und die Komponenten c) und d) in einer solchen Menge eingesetzt werden, die einem Äquivalentverhältnis von nach der Blockierungsreaktion noch vorliegenden freien Isocyanatgruppen zu Amino- und Hydroxylgruppen von 0,65 : 1 bis 1,05 : 1 entsprechen, wobei das Äquivalentverhältnis von Aminogruppen der Komponente c) zu Hydroxylgruppen der Komponente d) bei 1 : 0 bis 1 : 20 liegt, und wobei die Reaktionpartner a) bis d) in beliebiger Reihenfolge miteinander umgesetzt werden, wobei jedoch vorzugsweise die Komponente c) nicht vor den Komponenten b) und d) mit der Komponente a) zur Reaktion gebracht wird.

Gegenstand der Erfindung ist schließlich auch die Verwendung der Lackpolyisocyanate in Kombination mit organischen Polyhydroxylverbindungen als Einbrennlack oder zur Herstellung eines Einbrennlacks für beliebige hitzeresistente Substrate, insbesondere zur Drahtlackierung.

Bei der Durchführung des erfindungsgemäßen Verfahrens zur Herstellung der neuen Lackpolyisocyanate mit blockierten Isocyanatgruppen werden einfache Ausgangsdiisocyanate a) mit Blockierungsmitteln b), 1,3,5-Triazin-Derivaten c) und gegebenenfalls organischen Polyhydroxylverbindungen d) im Sinne einer Isocyanat-Additionsreaktion umgesetzt. "Blockierte Isocyanatgruppen" entstehen hierbei naturgemäß bei der Reaktion mit den gegenüber Isocyanatgruppen monofunktionellen Blockierungsmitteln b), während der unter (iii) benutzte Begriff "Urethangruppen" ausschließlich die durch Reaktion mit Polyhydroxylgruppen d) gebildeten Urethangruppen umfaßt.

Die Menge der Blockierungsmittel b) wird hierbei so bemessen, daß sie zur Blockierung von 20 - 90 %, vorzugsweise 35 - 80 % und besonders bevorzugt 50 - 70 % der Isocyanatgruppen der Ausgangsdiisocyanate a) ausreicht. Die Menge der Komponenten c) und d) wird so bemessen, daß das Äquivalentverhältnis der gegenüber den Blockierungsmitteln b) überschüssigen Isocyanatgruppen der Komponente a) zu den Amino- und Hydroxylgruppen der Komponenten c) und d) bei 0,65 : 1 bis 1,05 : 1, vorzugsweise 0,8 : 1 bis 1,0 : 1 liegt, wobei das Äquivalentverhältnis der Aminogruppen der Komponente c) zu den Hydroxylgruppen

der Komponente d) bei 1 : 0 bis 1 : 20, vorzugsweise 1 : 0 bis 1 : 4 und besonders bevorzugt bei 1 : 0 bis 1 : 1 liegt.

Als Ausgangsdiisocyanate a) eignen sich beliebige aromatische, aliphatische oder cycloaliphatische Diisocyanate des Molekulargewichtsbereichs 160 - 300 wie beispielsweise 1,4-Phenylendiisocyanat, 2,4- und 2,6-Diisocyanatotoluol (TDI) sowie beliebige Gemische dieser Isomeren, Diphenylenmethan-2,4′- und/oder -4,4′-diisocyanat, 1,5-Naphthylendiisocyanat, 2-Methylpentan-1,5-diisocyanat, 1,5-Hexandiisocyanat, 1,6-Hexandiisocyanat (HDI), 1,3- und 1,4-Cyclohexandiisocyanat sowie beliebige Gemische dieser Isomeren, 3,5,5-Trimethyl-3-isocyanatomethyl-cyclohexanisocyanat (IPDI) und Dicyclohexylmethan-2,4′- und/oder -4,4′-diisocyanat und beliebige Gemische dieser Diisocyanate.

Besonders bevorzugt einzusetzende Diisocyanate sind 2,4-Diisocyanatotoluol und 2,6-Diisocyanatotoluol, die aus diesen Isomeren bestehenden technischen Gemische und 4,4′-Diisocyanatodiphenylmethan und 2,4′-Diisocyanatodiphenylmethan, aus diesen Isomeren bestehende Gemische oder auch beliebige Gemische der hier genannten aromatischen Diisocyanate.

Als Blockierungsmittel b) sind die zur Blockierung von Isocyanatgruppen üblichen monofunktionellen Verbindungen geeignet, wie sie z.B. bei Z.W. Wicks, Progress in Organic Coatings 3 , 73 ff (1975) und 9 , 3 ff (1981) genannt sind. Bevorzugt sind Phenole, aliphatische Alkohole, Oxime und Lactame. Beispielhaft seien als Blockierungsmittel b) genannt: Phenol, Kresol, Xylenol sowie deren technische Isomerengemische, Isopropanol, Cyclohexanol, 1-Methoxy-2-propanol (MP), Diethylenglykolmonoethylether, Benzylalkohol, Butanonoxim, Cyclohexanonoxim und ε-Caprolactam, von denen Kresol, 1-Methoxy-2-propanol, Butanonoxim und ε-Caprolactam besonders bevorzugt sind.

Geeignete 1,3,5-Triazin-Derivate c) sind Verbindungen der Formel

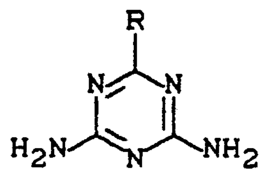

in welcher

R für einen gegenüber Isocyanatgruppen inerten organischen Rest, vorzugsweise einen $C_1$-$C_4$-Alkyl-, Phenyl- oder einen $C_1$-$C_6$-Alkoxyrest oder, besonders bevorzugt, für eine Aminogruppe steht.

Besonders bevorzugtes 1,3,5-Triazin-Derivat c) ist Melamin.

Als gegebenenfalls mitzuverwendende Polyhydroxylverbindungen d) kommen vorzugsweise Diole und/oder Triole des Molekulargewichtsbereichs 62 - 350, jedoch auch ein über 350 und bis 3.000 liegendes Molekulargewicht aufweisende höhermolekulare Polyhydroxylverbindungen in Betracht.

Geeignete Polyhydroxylverbindungen des Molekulargewichtsbereichs 62 - 350 sind beispielsweise Ethylenglykol, Propandiole, Butandiole, Hexandiole, Di-, Tri- oder Tetraethylenglykol, Di-, Tri- oder Tetrapropylenglykol, Neopentylglykol, 2-Ethyl-1,3-hexandiol, 2,2,4-Trimethyl- 1,3-pentandiol, 1,4-Bis(hydroxymethyl)-cyclohexan, 2,2-Bis(4-hydroxycyclohexyl)-propan, Trimethylolpropan, Glycerin, Hexantriol, N,N′,N″-Tris(2-hydroxyethyl)isocyanurat (THEIC) und Pentaerythrit.

Höhermolekulare Polyhydroxylverbindungen, die jedoch gegenüber den beispielhaft genannten niedermolekularen Polyhydroxylverbindungen keineswegs bevorzugt sind, sind beispielsweise die bekannten Polyhydroxypolyester, wie sie aus Dicarbonsäuren wie Phthalsäure, Isophthalsäure, Terephthalsäure oder Adipinsäure mit überschüssigen Polyolen der obengenannten Art erhalten werden. Als höhermolekulare Polyole können ebenfalls, jedoch auch weniger bevorzugt, an sich bekannte Polyhydroxypolyether, wie sie durch Alkoxylierung von niedermolekularen Startermolekülen zugänglich sind, verwendet werden.

Bei der Durchführung des erfindungsgemäßen Verfahrens können selbstverständlich auch jeweils Gemische der genannten Ausgangsmaterialien eingesetzt werden.

Bei der Durchführung des erfindungsgemäßen Verfahrens werden die Ausgangsmaterialien b), c) und gegebenenfalls d) in beliebiger Reihenfolge mit der Diisocyanat-Komponente a) umgesetzt, mit der einzigen Einschränkung, daß die Komponente c) vorzugsweise nicht vor den Komponenten b) und d) zum Einsatz gelangen. Dies bedeutet, daß beispielsweise a) mit einem Gemisch aus b), c) und d) einstufig umgesetzt werden kann oder aber auch, daß a) zunächst mit b) oder mit d) oder mit einem Gemisch aus b) und d) und anschließend mit c) bzw. mit Gemischen aus c) und b) bzw. aus c) und d) zur Reaktion gebracht werden kann.

Die genannten Umsetzungen werden im allgemeinen innerhalb des Temperaturbereichs von 20 - 200° C, vorzugsweise 60 - 180° C durchgeführt.

Gemäß einer bevorzugten Ausführungsform des erfindungsgemäßen Verfahrens wird das Ausgangsdiisocyanat a) mit dem Blockierungsmittel b) im angegebenen Mengenverhältnis blockiert. Die Blockierung erfolgt in Abhängigkeit von der Art des Blockierungsmittels im Temperaturbereich von 20 - 200 °C, bevorzugt bei 60 - 160 °C, bis der berechnete NCO-Gehalt erreicht oder geringfügig unterschritten ist. Nach beendeter Blockierung wird dann das Lösungsmittel zugegeben, sofern ein gelöstes Endprodukt hergestellt werden soll, und es wird in einer zweiten Reaktionsstufe die Umsetzung des teilblockierten Diisocyanats mit Melamin c), das gegebenenfalls im Gemisch mit mitverwendetem Polyol d) zugegeben wird, im Temperaturbereich von 60 - 180 °C, bevorzugt 80 - 160 °C, durchgeführt. Dabei reagiert in der Regel bei ca. 80 °C zunächst das gegebenenfalls mitverwendete Polyol d) und nachfolgend bei erhöhter Reaktionstemperatur, bevorzugt bei ca. 140 °C, das Melamin c). Die Umsetzung ist beendet, wenn eine klare Lösung entstanden ist, oder das lösungsmittelfreie Harz in z.B. Aceton klar löslich ist, und der Gehalt des Reaktionsgemischs an freien Isocyanatgruppen auf einen Wert von unter 0,5 Gew.-% abgefallen ist.

Nach dieser bevorzugten Verfahrensvariante lassen sich erfindungsgemäße Lackpolyisocyanate als Lösungen in Lacklösungsmitteln oder auch als lösungsmittelfreie Harze, die in der Regel als Festharz aus der Schmelze erstarren, herstellen.

Gemäß einer weiteren Ausführungsform des erfindungsgemäßen Verfahrens erfolgt die als erster Reaktionsschritt durchgeführte Blockierung bereits in Gegenwart eines geeigneten Lösungsmittels, worauf sich die Umsetzungen mit den anderen Reaktionspartnern entsprechend der soeben beschriebenen bevorzugten Ausführungsform anschließen.

Eine weitere Ausführungsform des erfindungsgemäßen Verfahrens kann beispielsweise in einer Eintopfreaktion bestehen, bei welcher Diisocyanat a), Blockierungsmittel b), Melamin c) und gegebenenfalls Polyol d) gemeinsam aufgeheizt werden, wobei im Bereich von 60 - 90 °C zunächst das aliphatische Polyol abreagiert, im Bereich von 90 - 120 °C das vorzugsweise eingesetzte phenolische Blockierungsmittel abreagiert und schließlich im Bereich von 120 - 140 °C die Umsetzung mit dem Melamin erfolgt. Die einzelnen Reaktionsstufen überlappen sich und können meist anhand der Exothermie mit abklingender Wärmetönung nach vollständigem Umsatz verfolgt werden.

Nur in außergewöhnlichen Fällen und keineswegs bevorzugt ist es auch möglich, zunächst das Diisocyanat a) mit dem Melamin c) und gegebenenfalls dem Polyol d) bis zum Erreichen des berechneten NCO-Gehalts umzusetzen und erst anschließend die Blockierung der verbleibenden Isocyanatgruppen mit dem Blockierungsmittel b) durchzuführen.

Die Durchführung des erfindungsgemäßen Verfahrens erfolgt in der Regel in Lösung, jedoch ist es in Abhängigkeit von den Eigenschaften des gewünschten Endproduktes, wie z.B. Verzweigungsgrad und Schmelzviskosität, durchaus auch möglich in Schmelze zu arbeiten.

Als Lösungsmittel geeignet sind alle an sich bekannten üblichen Lacklösemittel, die gegenüber Isocyanatgruppen inert sind, wie z.B. Ethylacetat, Butylacetat, Ethylenglykolmonomethyl- oder -ethyletheracetat, 1-Methoxypropyl-2-acetat, 2-Butanon, 4-Methyl-2-pentanon, Cyclohexanon, Toluol, Xylol, Solventnaphtha oder deren Gemische. Ebenfalls als Lösungsmittel verwendbar, jedoch weniger bevorzugt sind Weichmacher wie z.B. solche auf Basis von Phosphorsäure-, Sulfonsäure- oder Phthalsäureester Außer den gegenüber NCO-Gruppen inerten Lacklösemitteln ist es aber auch möglich, anteilig gegenüber NCO-Gruppen reaktive Lösemittel mitzuverwenden. Diese Lösemittel werden dann erst nach beendeter oder nahezu beendeter Umsetzung, z.B. bei einem erreichten NCO-Gehalt ≦ 0,5 %, zugegeben, was darauf hinausläuft, daß die im wesentlichen in Abwesenheit von derartigen Lösungsmitteln hergestellten Lackpolyisocyanate nach ihrer Herstellung in solchen Lösungsmitteln gelöst werden können. Geeignet sind bevorzugt monofunktionelle, aliphatische, cycloaliphatische, araliphatische Alkohole oder auch Phenole wie z.B. Isopropanol, n-Butanol, n-Octanol, 2-Methoxyethanol, 2-Ethoxyethanol, Diethylenglykolmonomethylether, Diethylenglykolmonoethylether, 1-Methoxy-2-propanol; Cycloalkanole wie Cyclopentanol oder Cyclohexanol; Aralkanole wie Benzylalkohol oder Phenole wie Kresol oder Xylenol.

Aus dem Umstand, daß die hier als Lösungsmittel genannten einwertigen Alkohole oder Phenole auch als Blockierungsmittel b) in Betracht kommen können, geht bereits hervor, daß bei Verwendung derartiger Alkohole oder Phenole als Lösungsmittel diese nicht in die Berechnung der Mengenverhältnisse der Reaktionspartner a) bis d) eingehen, sondern im wesentlichen erst im Anschluß an die erfindungsgemäße Umsetzung als Lösungsmittel zur Anwendung gelangen.

Die erfindungsgemäßen Lackpolyisocyanate stellen wertvolle Reaktionspartner für organische Polyhydroxylverbindungen dar und können in Kombination mit derartigen Polyhydroxylverbindungen als Einbrennlack oder zur Herstellung von Einbrennlacken für beliebige hitzeresistente Substrate verwendet werden. Geeignete Polyhydroxylverbindungen zur Herstellung von derartigen Lacken sind beispielsweise die nachstehend unter 1. bis 5. beispielhaft genannten Verbindungen bzw. beliebige Gemische derartiger Verbindungen:

1. Niedermolekulare, mehrwertige Alkohole mit einem maximalen Molekulargewicht von 350 und einer bei 2 bis 4 liegenden Hydroxylfunktionalität wie sie schon oben als Polyole d) beispielhaft aufgeführt wurden sowie aber z.B. auch 2,2-Bis[4-(2-hydroxyethoxy)-phenyl]propan, 2,2-Bis[4-(2-hydroxypropoxy)-phenyl]propan, Maleinsäure-bis-ethylenglykolester oder Adipinsäure-bis-ethylenglykolester.

2. Höhermolekulare Polyhydroxylpolyester des Molekulargewichtsbereichs 351 - 4.000, wie sie durch Umsetzung von mehrbasischen Carbonsäuren wie z.B. Adipinsäure, Phthalsäure, Tetrahydrophthalsäure, Hexahydrophthalsäure oder von deren Anhydriden mit überschüssigen Mengen an mehrwertigen Alkoholen der unter 1. beispielhaft genannten Art zugänglich sind.

3. Aliphatisch gebundene Hydroxylgruppen aufweisende Oligourethane des Molekulargewichtsbereichs 200 -2.000, wie sie durch Umsetzung der bereits unter 1. genannten, gegebenenfalls Ethergruppen aufweisenden Alkandiole oder -triole mit unterschüssigen Mengen an Diisocyanaten der auch als erfindungsgemäße Ausgangsmaterialien geeigneten und oben unter a) genannten Art erhalten werden und wie sie beispielsweise in DE-PS 1 644 794 oder in GB-PS 1 195 886 beschrieben sind.

4. Hydroxylgruppenhaltige Copolymerisate, wie sie durch Copolymerisation von z.B. Hydroxyalkylacrylaten bzw. -methacrylaten mit Acryl- bzw Methacrylsäurealkylestern, sowie gegebenenfalls weiteren olefinisch ungesättigten Monomeren und/oder wie sie gemäß der DE-OS 2 137 239 aus Styrol-Maleinsäure-Copolymerisaten durch teilweise Veresterung der Säuregruppen mit Ethylenoxid erhalten werden.

5. Hydroxylgruppen-aufweisende Polycarbonate der an sich bekannten Art, wie sie z.B. durch Umsetzung von Diolen wie 1,3-Propandiol, 1,4-Butandiol und/oder 1,6-Hexandiol, Di-, Tri- und/oder Tetraethylenglykol mit Diarylcarbonaten, z.B. Diphenylcarbonat, oder Phosgen hergestellt werden können, vorzugsweise Polycarbonate auf Basis von 1,6-Hexandiol sowie gegebenenfalls modifizierend wirkende Co-Diole in untergeordneten Mengen (ca. 5 - 35 Mol-% der Diole). Auch Polycarbonate auf Basis von $HO(CH_2)_6$-O-CO-$(CH_2)_5$-OH kommen in Frage.

Die unter 2. und 3. beispielhaft genannten Verbindungen sind die bevorzugten Reaktionspartner für die erfindungsgemäßen Lackpolyisocyanate mit blockierten Isocyanatgruppen bei der Herstellung von Einbrennlacken. Neben den beispielhaft genannten Reaktionspartnern können in den Einbrennlacken jedoch auch noch weitere gegenüber Isocyanatgruppen reaktionsfähige Verbindungen vorliegen, obwohl dies im allgemeinen weniger bevorzugt ist. In diesem Zusammenhang sind z.B. Hydroxylgruppen aufweisende oligomere Epoxide, Imidester, Imidesteramide, Hydantoine oder Aminogruppen aufweisende Verbindungen wie Hexamethylendiamin, Melamin/Formaldehydharze oder Aminopolyether zu nennen.

Durch Wahl des Molekulargewichts der einzusetzenden Polyhydroxylverbindungen können die makroskopischen Eigenschaften des bei der erfindungsgemäßen Verwendung entstehenden Flächengebildes auf einfache Weise variiert werden. So führt im allgemeinen die Verwendung von höhermolekularen Polyhydroxylverbindungen zu elastischeren, die Verwendung von niedermolekularen Polyhydroxylverbindungen zu härteren Beschichtungen.

Bei der erfindungsgemäßen Verwendung werden die erfindungsgemäßen Lackpolyisocyanate mit den beispielhaft genannten Polyhydroxylverbindungen in solchen Mengenverhältnissen kombiniert, daß auf jede blockierte Isocyanatgruppe der erfindungsgemäßen Lackpolyisocyanate 0,1 - 10, vorzugsweise 0,2 - 2, besonders bevorzugt 0,5 - 1,2, Hydroxylgruppen der Hydroxylgruppen-aufweisenden Reaktivkomponente entfallen.

Die Kombinationen der erfindungsgemäßen Lackpolyisocyanate mit den beispielhaft genannten Polyhydroxylverbindungen können als solche als Einbrennlack Verwendung finden. Sie können jedoch auch noch mit Hilfs- und Zusatzmitteln wie beispielsweise Lösungsmitteln, Katalysatoren, Pigmenten und dergleichen abgemischt werden.

Geeignete Lösungsmittel sind insbesondere die bereits oben beispielhaft genannten Lösungsmittel. Im Falle der Verwendung von einwertigen Alkoholen oder Phenolen als Lösungsmittel werden diese während des Einbrennvorgangs durch die nicht flüchtigen Polyhydroxylverbindungen verdrängt, so daß sie zusammen mit dem Blockierungsmittel aus dem Reaktionsgemisch während des Einbrennvorgangs abdestillieren. Für die erfindungsgemäß bevorzugte Drahtlackierung werden im allgemeinen 15- bis 75-, insbesondere 20- bis 60-gew.-%ige Lösungen der Kombinationen aus Lackpolyisocyanat und Polyhydroxylverbindungen eingesetzt.

Beispiele von erfindungsgemäß zu verwendenden Katalysatoren sind im Kunststoffhandbuch (Hrsg. Becker/Braun), Bd. 7, Polyurethane, S. 92 ff, Carl Hanser Verlag, München Wien 1983, beschrieben. Geeignet sind beispielsweise auch die in DE-AS 2 626 175, Kolonne 7, Zeile 35 bis Kolonne 8, Zeile 27, beschriebenen Katalysatoren. Besonders geeignet sind organische Metallkatalysatoren wie insbesondere organische Titan-, Zink- oder Zinnverbindungen wie Tetraisopropyltitanat, Zinkoctoat, Dibutylzinnoxid oder Dibutylzinndilaurat.

Die Katalysatoren werden, falls überhaupt, in einer Menge von 0,01 bis 5,0, vorzugsweise 0,1 bis 3,0 Gew.-%, bezogen auf die erfindungsgemäßen blockierten Polyisocyanate eingesetzt.

Die durch Abmischung bei Raumtemperatur aus den genannten erfindungswesentlichen Komponenten bzw. Hilfs- und Zusatzmitteln erhaltenen Gemische sind bei Raumtemperatur oder mäßig erhöhter Temperatur (bis ca. 50 °C) lagerbeständig und reagieren beim Erhitzen auf Temperaturen von oberhalb 60 °C, vorzugsweise von 100 - 500 °C und insbesondere von 180 - 400 °C unter gleichzeitigem Verdampfen der gegebenenfalls vorliegenden flüchtigen Bestandteile (z.B. Lösungsmittel) zu vernetzten Kunststoffen aus.

Die unter erfindungsgemäßer Verwendung der erfindungsgemäßen Lackpolyisocyanate hergestellten Einbrennlacke eignen sich zur Lackierung von beliebigen hitzeresistenten Substraten, insbesondere von Glasfasern und besonders bevorzugt von Metalldrähten. Die aus Kombinationen der erfindungsgemäßen Lackpolyisocyanate mit organischen Polyhydroxylverbindungen oder aus Abmischungen derartiger Kombinationen mit Hilfs- und Zusatzmitteln der genannten Art bestehenden Einbrennlacke können nach allen Methoden der Beschichtungstechnologie auf die Substrate aufgetragen werden, worauf sich eine Aushärtung der Beschichtung innerhalb der oben genannten Temperaturbereiche anschließt.

Bei der bevorzugten Drahtlackierung erfolgt die Beschichtung der Drähte nach den an sich bekannten Tauch-, Rollenauftrags- oder Saugfilzverfahren, worauf sich die Trocknung, d.h die Aushärtung der Lackschichten in den üblichen Trockenöfen innerhalb der genannten Temperaturbereiche anschließt. Bei der Verwendung der erfindungsgemäßen Produkte als Vernetzer für Drahtlackierungen sind hier insbesondere die hervorragende Haftung auf dem Draht sowie die mögliche hohe Lackiergeschwindigkeit im Vergleich zu herkömmlichen bekannten Produkten des Standes der Technik hervorzuheben.

Wegen der ausgezeichneten elektrischen und mechanischen Eigenschaften der aus den erfindungsgemäßen Lack-Polyisocyanaten und den beispielhaft genannten Polyhydroxylverbindungen unter Hitzeeinwirkung entstehenden Kunststoffe eignen sich die genannten Kombinationen aus erfindungsgemäßen Lackpolyisocyanaten und beispielhaft genannten Polyhydroxylverbindungen bzw. die Abmischungen dieser Kombinationen mit den beispielhaft genannten Hilfs- und Zusatzmitteln auch zur Herstellung von Isoliergeweben oder zur Imprägnierung von Elektromotoren.

In den nachfolgenden Beispielen beziehen sich alle Prozentangaben auf Gewichtsprozente. Die Gehalte an blockierten NCO-Gruppen wurden als "NCO", d.h. unter Zugrundelegung eines Molekulargewichts von 42 berechnet. Die Gehalte an Urethangruppen beziehen sich ausschließlich auf die durch Reaktion von Isocyanatgruppen mit mehrwertigen Alkoholen gebildeten Urethangruppen und umfassen demzufolge nicht die aus Isocyanatgruppen und einwertigen alkoholischen oder phenolischen Blockierungsmitteln gebildeten blockierten Isocyanatgruppen, die chemisch selbstverständlich ebenfalls Urethangruppen darstellen.

## Beispiele

### Beispiel 1

#### a) Herstellung

Zu 2.064 Gewichtsteilen Diisocyanatotoluol (2,4- und 2,6-Isomere im Gewichtsverhältnis 8 : 2) werden bei 80 °C beginnend 1.537 Gewichtsteile Kresol (technisches Isomerengemisch) unter Aufheizen bis 130 °C innerhalb ca. 1 Stunde zugetropft. Man läßt ca. 8 Stunden bis 15 Stunden bei 120 - 140 °C reagieren, bis ein NCO-Gehalt von 11,0% erreicht ist ($NCO_{theor.}$ = 11,1 %). Man löst unter Abkühlen durch Zugabe von 5.000 Gewichtsteilen 1-Methoxypropyl-2-acetat und fügt bei 80 °C eine Suspension von 399 Gewichtsteilen Melamin in 1.000 Gewichtsteilen 1-Methoxypropyl-2-acetat zu. Man heizt rasch unter intensivem Rühren der Suspension bis 140 °C auf. Nach ca. 3 Stunden Reaktionszeit wird eine klare Lösung erhalten; nach 1 Stunde Nachrühren ist die Umsetzung beendet, der freie NCO-Gehalt beträgt < 0,1 %.

Man erhält eine klare Lösung des blockierten Isocyanats mit folgenden Kenndaten (alle Angaben beziehen sich hier und in den folgenden Herstellungsbeispielen auf die

| Lösung): | |
|---|---|
| Konzentration:<br>Viskosität $_\eta$(23 °C):<br>freier NCO-Gehalt:<br>blockierter NCO-Gehalt: | 40 %<br>28.000 mPas<br>< 0,1 %<br>5,9 % |
| (Analysenmethode: 30 min/180 °C mit Di-n-butylamin in o-Dichlorbenzo) | |
| blockierter NCO-Gehalt (ber.):<br>1,3,5-Triazineinheiten (ber.):<br>Urethangruppen (ber.): | 6,0 %<br>2,5 %<br>0 % |

b) Verwendung

200 Gewichtsteile eines Polyesters mit der OH-Zahl 395, hergestellt aus 40,8 % Trimethylolpropan, 14,2 % Ethylenglykol und 45,0 % Phthalsäureanhydrid, werden in 800 Gewichtsteilen eines Lösemittelgemisches aus gleichen Gewichtsteilen Kresol und Xylol unter Rückfluß bei Temperaturen bis 130 °C gelöst. Zu der erhaltenen Lösung werden 1.000 Gewichtsteile der wie unter a) beschrieben hergestellten 40 %igen Lösung des blockierten Polyisocyanats und 6 Gewichtsteile eines Aldimins auf Basis Butyraldehyd und Anilin als Katalysator gegeben.

Mit der so hergestellten Lösung wird ein Kupferdraht von 0,7 mm Durchmesser lackiert.

| Lackierofen: | vertikal mit Filzabstreifer und 6 Durchzügen |
|---|---|
| Ofenlänge:<br>Ofentemperatur:<br>Durchmesserzunahme durch die Lackierung:<br>Lackiergeschwindigkeit: | 4 m<br>350 °C<br>40 - 50 µm<br>10 - 16 m/min |

Der so erhaltene Lackdraht läßt sich bei 320 °C innerhalb 9 sec verzinnen und hat eine Bleistifthärte von 4 H, die auch nach halbstündigem Eintauchen in auf 60 °C erwärmtes Ethanol unverändert gegeben ist.

Bei Verwendung eines herkömmlichen, handelsüblichen Lacksystems, das sich lediglich bezüglich der Natur des Härters vom dem beschriebenen erfindungsgemäßen Lacksystem unterscheidet [es wurde eine äquivalente Menge eines blockierten Isocyanats, bestehend aus dem Umsetzungsprodukt von 55,6 % Diisocyanatotoluol (2,4- und 2-6-Isomere im Gewichtsverhältnis 8 : 2), 14,3 % Trimethylolpropan und 30,1 % Phenol verwendet] läßt sich der erhaltene Lackdraht bei 320 °C überhaupt nicht oder erst nach wesentlich längerer Zeit als 9 sec verzinnen. Die Bleistifthärte fällt nach der halbstündigen Ethanolbehandlung bei 60 °C auf den Wert HB ab.

Beispiel 2

a) Herstellung

Zu 2.172 Gewichtsteilen Diisocyanatotoluol (2,4- und 2,6-Isomere im Gewichtsverhältnis 8 : 2) wird innerhalb ca. 30 min bei 120 °C eine Lösung von 1.408 Gewichtsteilen Phenol in 3.000 Gewichtsteilen 1-Methoxypropyl-2-acetat zugetropft. Man läßt ca. 2 Stunden bei 120 °C reagieren und läßt nach Erreichen eines NCO-Gehaltes von 6,7 % ($NCO_{theor.}$ = 6,4 %) abkühlen. Bei 40 °C und einem NCO-Gehalt der Lösung von jetzt 6,3 % wird eine Suspension von 419 Gewichtsteilen Melamin in weiteren 3.000 Gewichtsteilen 1-Methoxypropyl-2-acetat zufließen gelassen. Man heizt unter Rühren bis 140 °C. Nach ca.

1,5 Stunden wird die Mischung klar und man beendet die Umsetzung nach 1 Stunde Nachrühren bei 140 °C.

Man erhält eine klare hellgelb gefärbte Lösung mit folgenden Kenndaten:

| | |
|---|---|
| Konzentration: | 40 % |
| Viskosität „(23 °C): | 3.240 mPas |
| freier NCO-Gehalt: | 0,4 % |
| blockierter NCO-Gehalt (gef.): | 6,6 % |
| blockierter NCO-Gehalt (ber.): | 6,3 % |
| 1,3,5-Triazineinheiten (ber.): | 2,6 % |
| Urethangruppen (ber.): | 0 % |

b) Verwendung

150 Gewichtsteile eines Hydroxyurethans der OH-Zahl 190, hergestellt aus 26,5 % 1,6-Hexandiol, 21,0 % Trimethylolpropan, 1,8 % ε-Caprolactam und 50,7 % Diisocyanatotoluol (2,4- und 2,6-Isomere im Verhältnis 8 : 2), werden bei 130 °C unter Rückfluß in 475 Gewichtsteilen einer Mischung aus gleichen Gewichtsteilen Kresol und Xylol gelöst und nach Erkalten mit 375 Gewichtsteilen der, wie unter a) beschrieben, hergestellten Lösung des blockierten Polyisocyanats versetzt. Als Katalysator wurden 3 Gewichtsteile eines Aldimins auf Basis von Butyraldehyd und Anilin (1 % bezogen auf FK) zugesetzt.

Auf einer vertikalen Drahtlackieranlage mit einem Ofen von 4 m Länge wird ein Kupferdraht von 0,7 mm Durchmesser mittels Filzabstreifer in 6 Durchzügen auf eine Durchmesserzunahme von 50 µm lackiert. Bei einer Ofentemperatur von 350 °C kann in einem Fahrbereich von 10 - 20 m/min lackiert werden, ohne daß beim Dehnen des Cu-Drahtes bis zum Bruch der Lackfilm Risse zeigt. Der erhaltene Lackdraht hat eine bemerkenswert gute Abrieb festigkeit und läßt sich bei 320 °C verzinnen. (Bei Prüfung der Abriebfestigkeit nach der NEMA-Methode werden bei einem Auflagegewicht von 400 p > 100 Doppelhübe ausgehalten.)

Bei Verwendung des in Beispiel 1 erwähnten herkömmlichen Härters durch äquivalenten Austausch des erfindungsgemäßen Polyisocyanates läßt sich der bei sonst analogen Lackierbedingungen erhaltene Lackdraht bei 320 °C nicht verzinnen, oder die Verzinnungszeit liegt je nach Einbrenngrad deutlich oberhalb 10 sec. Die Abriebfestigkeit ist bedeutend schlechter; es werden etwa nur die Hälfte der Doppelhübe, wie oben beschrieben, ausgehalten.

Beispiel 3

a) Herstellung

2.589 Gewichtsteile Kresol (technisches Isomerengemisch) werden bei 80 °C beginnend unter Aufheizen bis 130 °C zu 2.979 Gewichtsteilen Diisocyanatotoluol (2,4- und 2,6-Isomere im Gewichtsverhältnis 8 : 2) rasch zufließen gelassen. Nach ca. 12 Stunden wird ein Isocyanatgehalt von 7,5 % erreicht, und man löst durch Zugabe von 3.000 Gewichtsteilen 1-Methoxypropyl-2-acetat. Unterhalb 80 °C läßt man eine Suspension von 432 Gewichtsteilen Melamin in weiteren 1.000 Gewichtsteilen 1-Methoxypropyl-2-acetat zufließen. Nach Aufheizen bis 140 °C wird die Suspension im Laufe der Umsetzung nach ca. 2,5 Stunden klar, und man beendet die Umsetzung nach ca. 1 Stunde Nachrührzeit, wenn der NCO-Gehalt auf Null abgesunken ist.

Man erhält ein klar gelöstes Produkt mit folgenden Kenndaten:

| | |
|---|---|
| Konzentration: | 60 % |
| Viskosität „(23 °C): | 20.000 mPas |
| freier NCO-Gehalt: | < 0,1 % |
| blockierter NCO-Gehalt (gef.): | 9,9 % |
| blockierter NCO-Gehalt (ber.): | 10,1 % |
| 1,3,5-Triazineinheiten (ber.): | 2,7 % |
| Urethangruppen (ber.): | 0 % |

b) Verwendung

150 Gewichtsteile des in Beispiel 2b) beschriebenen Hydroxyurethans werden in 600 Gewichtsteilen eines Gemisches aus gleichen Gewichtsteilen Kresol und Xylol unter Rückfluß bei Temperaturen bis 130 °C gelöst. Die erhaltene Lösung wird mit 250 Gewichtsteilen der wie oben unter a) beschrieben hergestellten Lösung des blockierten Isocyanats vermischt. Anschließend werden der so erhaltenen Lackmischung 3 Gewichtsteile eines Aldimins auf Basis Butyraldehyd/Anilin als Katalysator (1 %. bezogen auf FK) zugegeben.

Auf einer Drahtlackiermaschine mit einem Einbrennschacht von 4 m Länge wird ein Kupferdraht von 0,7 mm Durchmesser mittels Abstreiferfilz in 6 Durchzügen auf eine Durchmesserzunahme von 50 µm lackiert. Bei einer Temperatur des Einbrennschachtes von 400 °C kann die Durchzugsgeschwindigkeit des Drahtes von 8 m/min bis 20 m/min variiert werden. Innerhalb dieses Einbrennbereichs wird ein einwandfreier lackierter Draht erhalten.

Bei der Prüfung auf Wasserbruch (Haarrisse) verhält sich der Lackdraht zufriedenstellend; er kann unter Wasser um einen Dorn von 20 mm Durchmesser gebogen werden, ohne daß Haarrisse auftreten. Zur Prüfung biegt man den Draht in einer 0,5 %igen NaCl-Lösung um einen enger werdenden Dorn bei 100 mm Durchmesser beginnend und legt an das Wasserbad und den Kupferleiter eine Gleichspannung von 100 V an; es wird kein Stromfluß beobachtet.

Beispiel 4

a) Herstellung

3.085 Gewichtsteile Diisocyanatotoluol (2,4- und 2,6-Isomere im Gewichtsverhältnis 8 : 2) werden wie in Beispiel 1a) beschrieben mit 2.298 Gewichtsteilen Kresol (technisches Isomerengemisch) blockiert. Nach Erreichen eines NCO-Gehaltes von 11,1 % ($NCO_{theor}$ = 11,1 %) werden 4.000 Gewichtsteile 1-Methoxypropyl-2-acetat als Lösungsmittel zugegeben. Man fügt zu der auf 60 °C abgekühlten klaren Lösung eine Mischung aus 298 Gewichtsteilen Melamin und 319 Gewichtsteilen 1,3-Butandiol zu und läßt 2 Stunden bei 80 °C reagieren. Anschließend wird die noch heterogene Reaktionsmischung auf 140 °C erhitzt, und die Umsetzung wird bei dieser Temperatur bis zum Erreichen einer klaren Lösung beendet.

| | |
|---|---|
| Konzentration: | 60 % |
| Viskosität „(23 °C): | 8.800 mPas |
| freier NCO-Gehalt: | 0,2 % |
| blockierter NCO-Gehalt (ber.): | 8,9 % |
| 1,3,5-Triazineinheiten (ber.): | 1,8 % |
| Urethangruppen (ber.): | 4,2 % |

b) Verwendung

150 Gewichtsteile des Hydroxyurethans der OH-Zahl 190 wie in Beispiel 2b) beschrieben werden unter Erhitzen in 600 Gewichtsteilen einer Mischung aus gleichen Gewichtsteilen N-Methylpyrrolidon und 1-Methoxypropyl-2-acetat gelöst. Die erhaltene Lösung wird mit 250 Gewichtsteilen der wie oben unter a) beschrieben hergestellten Lösung vermischt, und man fügt 3 Gewichtsteile eines Aldimins auf Basis Butyraldehyd/Anilin als Katalysator zu.

Auf einer Drahtlackiermaschine mit einem 4 m langen Einbrennschacht bei 400 °C Ofentemperatur wird ein Kupferdraht von 0,7 mm Durchmesser mittels Abstreiferfilz in 6 Durchzügen auf eine Durchmesserzunahme von 50 µm lackiert.

Der so hergestellte Lackdraht besitzt eine hervorragende Haftfestigkeit. Bei der Prüfung nach DIN 46 453 8.4 werden Verdrehungszahlen von mehr als 500 erzielt, ehe erste Lackschichtablösungen auftreten.

Zum Vergleich wird ein Draht unter gleichen Bedingungen wie oben beschrieben lackiert, wobei jedoch das erfindungsgemäße blockierte Polyisocyanat aus Beispiel 4a) durch eine äquivalente Menge des in Beispiel 1b) beschriebenen herkömmlichen, handelsüblichen Produkts ausgetauscht wurde. Bei der Haftfestigkeitsprüfung entsprechend DIN 46 453 8.4 lassen sich nur Verdrehungszahlen bis ca. 350 erzielen. Die Haftfestigkeit des erfindungsgemäßen Lacks ist wesentlich besser.

## Beispiel 5

### a) Herstellung

Eine Diisocyanatmischung aus 1.197 Gewichtsteilen Diisocyanatotoluol (2,4- und 2,6-Isomere im Gewichtsverhältnis 8 : 2) und 1.719 Gewichtsteilen 4,4′-Diisocyanatodiphenylmethan wird bei 80 °C beginnend unter Heizen bis 130 °C mit 1.486 Gewichtsteilen Kresol (technisches Isomerengemisch) versetzt. Nach ca. 8 Stunden bei 130 °C und Erreichen eines NCO-Gehaltes von 13,1 % ($NCO_{theor.}$ = 13,1 %) werden 5.000 Gewichtsteile 1-Methoxypropyl-2-acetat zugegeben, und man versetzt bei 60 °C mit einer Mischung aus 289 Gewichtsteilen Melamin und 309 Gewichtsteilen 1,3-Butandiol. Man läßt 2 Stunden bei 80 °C und 2 Stunden bei 140 °C reagieren bis eine klare Lösung erreicht wird.

Die klare hellgelbe Lösung hat folgende Kenndaten:

| | |
|---|---|
| Konzentration: | 50 % |
| Viskosität „(23 °C): | 17.500 mPas |
| freier NCO-Gehalt: | < 0,1 % |
| blockierter NCO-Gehalt (ber.): | 5,8 % |
| 1,3,5-Triazineinheiten (ber.): | 1,8 % |
| Urethangruppen (ber.): | 4,1 % |

### b) Verwendung

150 Gewichtsteile des in Beispiel 2b) beschriebenen Hydroxyurethans werden in 550 Gewichtsteilen einer Mischung aus gleichen Gewichtsteilen N-Methylpyrrolidon und 1-Methoxypropyl-2-acetat unter Erhitzen bis 130 °C gelöst. Zu der erkaltetan Lösung werden 300 Gewichtsteile der wie oben unter a) beschrieben hergestellten Lösung des blockierten Polyisocyanats sowie 3 Gewichtsteile eines Katalysators, eines Aldimins auf Basis von Butyraldehyd/Anilin, zugegeben.

Auf einer Drahtlackieranlage mit 4 m langem Einbrennschacht wird ein Kupferdraht von 0,7 mm Durchmesser in 6 Durchzügen mittels Abstreiferfilz bei einer Ofentemperatur von 400 °C auf eine Durchmesserzunahme von 50 µm lackiert.

Man erhält einen Lackdraht mit ausgezeichneter Haarrißbeständigkait. Nach dem in Baispiel 3b) beschriebenen Dornbiegetest läßt sich der Lackdraht um einen Dorn von 20 mm biegen, ohne daß Haarrisse auftreten.

## Beispiel 6

a) Herstellung

Zu einer auf 80 °C geheizten Mischung aus 2.046 Gewichtsteilen 1-Methoxy-2-propanol, 339 Gewichtsteilen Trimethylolpropan und 318 Gewichtsteilen Melamin werden innerhalb ca. 20 min 3.297 Gewichtsteile Diisocyanatotoluol (2,4- und 2,6-Isomere im Gewichtsverhältnis 8 : 2) so zugetropft, daß die Temperatur bei exothermer Reaktion unterhalb 100 °C gehalten wird. Die viskoser werdende Reaktionsmischung wird nach ca. 1 Stunde bis 110 °C, nach ca. 1,5 Stunden bis 140 °C geheizt. Nach ca. 2,5 Stunden ist kein freies Isocyanat mehr nachweisbar, und man gibt als Lösungsmittel 4.000 Gewichtsteile 1-Methoxy-2-propanol zu. Man erhält eine klare farblose Lösung des mit Methoxypropanol blockierten Polyisocyanats mit folgenden Kenndaten:

| | |
|---|---|
| Konzentration: | 60 % |
| Viskosität „(23 °C): | 2.800 mPas |
| freir NCO-Gehalt: | NCO-frei |
| blockierter NCO-Gehalt (ber.): | 9,5 % |
| 1,3,5-Triazineinheiten (ber.): | 2,0 % |
| Urethangruppen (ber.): | 4,5 % |

b) Verwendung

150 Gewichtsteile des in Beispiel 2b) beschriebenen Hydroxyurethans werden in 600 Gewichtsteilen eines Lösemittelgemisches aus gleichen Gewichtsteilen N-Methylpyrrolidon und 1-Methoxypropyl-2-acetat unter Erwärmen auf 130 °C gelöst. Die erkaltete Lösung wird mit 250 Gewichtsteilen der wie unter a) beschrieben hergestellten Lösung des blockierten Polyisocyanats vermischt und mit 3 Gewichtsteilen Zinkoctoat (Zn-Gehalt = 8 %) als Katalysator (1 % bezogen auf FK) versetzt.

Auf einer vertikalen Drahtlackieranlage mit Filzabstreifern wird ein Kupferdraht von 0,7 mm Durchmesser in 6 Durchzügen auf eine Durchmesserzunahme von ca. 50 µm lackiert. Die Ofenschachttemperatur betrug 400 °C. Die Drahtlackiergeschwindigkeit wurde variiert von 12 m/min bis 16 m/min.

Bei dem erhaltenen lackierten Draht ist die Hitzeschockfestigkeit bis zu einer Temperatur von 260 °C gegeben und zeigt damit ausgezeichnete Werte.

Die Hitzeschockprüfung erfolgt nach DIN 46 453 durch Wickeln des Lackdrahtes um einen Dorn des eigenen Durchmessers (0,7 mm), so daß 10 aneinanderliegende Windungen entstehen. Drei derartige Wendel werden 30 min in einem auf 260 °C vorgeheizten Schrank gelagerte. Der Lackfilm der vorher einwandfreien Wendel weist danach keinerlei Risse oder Brüche auf.

Beispiel 7

a) Herstellung

In 2.000 Gewichtsteilen 1-Methoxypropyl-2-acetat werden 1.782 Gewichtsteile Butanonoxim, 215 Gewichtsteile Mela min und 384 Gewichtsteile 1,3-Butandiol vorgelegt. Innerhalb ca. 1 Stunde wird bei 60 °C beginnend eine Lösung von 1.485 Gewichtsteilen Diisocyanatotoluol (2,4- und 2,6-Isomere im Gewichtsverhältnis 8 : 2) und 2.134 Gewichtsteilen 4,4′-Diisocyanatodiphenylmethan in 2.000 Gewichtsteilen 1-Methoxypropyl-2-acetat zugetropft. Die zunächst exotherme Reaktionsmischung wird ca. 2 Stunden bei 80 °C und dann 1 Stunde bei 140 °C gerührt, bis kein freies Isocyanat mehr vorhanden ist und eine klare Lösung entstanden ist.

Die klare gelb gefärbte Lösung hat folgende Kenndaten:

| | |
|---|---|
| Konzentration: | 60 % |
| Viskosität „(23 ° C): | 4.320 mPas |
| freier NCO-Gehalt: | NCO-frei |
| blockierter NCO-Gehalt (ber.): | 8,6 % |
| 1,3,5-Triazineinheiten (ber.): | 1,3 % |
| Urethangruppen (ber.): | 5,0 % |

b) Verwendung

150 Gewichtsteile des in Beispiel 2b) beschriebenen Hydroxyurethans werden in 600 Gewichtsteilen eines Lösemittelgemisches aus gleichen Gewichtsteilen N-Methylpyrrolidon und 1-Methoxypropyl-2-acetat unter Erwärmen auf 130 ° C gelöst und nach dem Erkalten mit 250 Gewichtsteilen der wie unter a) beschrieben hergestellten Lösung des blockierten Polyisocyanates versetzt. Man gibt 3 Gewichtsteile Zinkoctoat (Zn-Gehalt = 8 %) als Katalysator zu.

Wie in Beispiel 6b) beschrieben wird ein Kupferdraht mit 0,7 mm Durchmesser unter gleichen Einbrennbedingungen lackiert. Die Drahtlackiergeschwindigkeit wird dabei von 10 bis 16 m/min variiert.

Die Hitzeschockfestigkeit (Prüfung wie in Beispiel 6b) beschrieben) ist bis 260 ° C gegeben und zeigt damit ebenfalls ausgezeichnete Werte.

Beispiel 8

Verwendung

230 Gewichtsteile der 65 %igen Lösung in 1-Methoxypropyl-2-acetat eines Hydroxyurethans, hergestellt aus 22,1 % Trimethylolpropan, 8,7 % Diethylenglykol, 7,4 % 1,3-Butandiol und 61,8 % 4,4'-Diisocyanatodiphenylmethan werden mit 395 Gewichtsteilen einer Mischung aus gleichen Gewichtsteilen Kresol und Xylol versetzt. Dazu gibt man 375 Gewichtsteile der wie in Beispiel 1a) beschrieben hergestellten Lösung des blockierten Polyisocyanats zu, sowie als Katalysator 3 Gewichtsteile eines Aldimins aus Butyraldehyd/Anilin.

Auf der in den vorausgehenden Beispielen beschriebenen Drahtlackieranlage wird bei einer Ofentemperatur von 400 ° C ein Kupferdraht von 0,7 mm Durchmesser in 6 Durchzügen mittels Filzabstreifer auf eine Durchmesserzunahme von 50 µm lackiert. Die Lackiergeschwindigkeit konnte dabei von 10 - 22 m/min variiert werden. Der erhaltene Lackdraht zeichnet sich durch eine besonders hohe Abriebfestigkeit aus. Bei einem Auflagegewicht von 380 p hält er 120 Doppelhübe aus (Prüfung wie in Beispiel 2b) beschrieben). Außerdem weist der Lackdraht eine gute Erweichungstemperatur auf; die Prüftemperatur von 250 ° C wird ausgehalten (DIN 46 453 10.1).

## Ansprüche

1. Lackpolyisocyanate mit blockierten Isocyanatgruppen, gekennzeichnet durch das gleichzeitige Vorliegen von

(i) 0,5 - 15 Gew.-% an über Harnstoffgruppen eingebauten Struktureinheiten der Formel

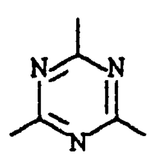 ,

(ii) 5 - 20 Gew-% an Isocyanatgruppen (berechnet als -NCO) in mit Blockierungsmitteln für Isocyanatgruppen blockierter Form und

(iii) 0 - 30 Gew.-% an sonstigen, von den blockierten Isocyanatgruppen verschiedenen, chemisch eingebauten Urethangruppen der Formel

-NH-CO-O- .

2. Verfahren zur Herstellung von Lackpolyisocyanaten mit blockierten Isocyanatgruppen gemäß Anspruch 1, dadurch gekennzeichnet, daß man

a) organische Diisocyanate des Molekulargewichtsbereichs 160 - 300 mit

b) Blockierungsmitteln für Isocyanatgruppen,

c) Aminogruppen aufweisenden Triazinen der Formel

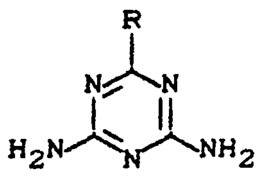

in welcher

R für eine Aminogruppe oder einen gegenüber Isocyanatgruppen inerten Substituenten steht und gegebenenfalls

d) organischen Polyhydroxylverbindungen des Molekulargewichtsbereichs 62 - 3.000

umsetzt, wobei die Menge des Blockierungsmittels b) in einer zur Blockierung von 20 - 90 % der Isocyanatgruppen der Komponente a) ausreichenden Menge und die Komponenten c) und d) in einer solchen Menge eingesetzt werden, die einem Äquivalentverhältnis von nach der Blockierungsreaktion noch vorliegenden freien Isocyanatgruppen zu Amino- und Hydroxylgruppen von 0,65 : 1 bis 1,05 : 1 entsprechen, wobei das Äquivalentverhältnis von Aminogruppen der Komponente c) zu Hydroxylgruppen der Komponente d) bei 1 : 0 bis 1 : 20 liegt, und wobei die Reaktionpartner a) bis d) in beliebiger Reihenfolge miteinander umgesetzt werden.

3. Verfahren gemäß Anspruch 2, dadurch gekennzeichnet, daß man die Blockierungsmittel b) in einer solchen Menge verwendet, die zur Blockierung von 35 - 80 % der Isocyanatgruppen der Komponente a) ausreicht, und die Komponenten c) und d) in einer solchen Menge verwendet, die einem Äquivalentverhältnis von nach der Blockierungsreaktion noch vorliegenden unblockierten Isocyanatgruppen zu Amino- und Hydroxylgruppen von 0,8 : 1 bis 1 : 1 entspricht, wobei das Äquivalentverhältnis von Aminogruppen der Komponente c) zu Hydroxylgruppen der Komponente d) bei 1 : 0 bis 1 : 4 liegt.

4. Verfahren gemäß Anspruch 2 und 3, dadurch gekennzeichnet, daß man als Komponente b) Blockierungsmittel, ausgewählt aus der Gruppe bestehend aus Phenolen, aliphatischen Alkoholen, Oximen und Lactamen verwendet.

5. Verfahren gemäß Anspruch 2 bis 4, dadurch gekennzeichnet, daß man als Komponente c) Melamin verwendet.

6. Verfahren gemäß Anspruch 2 bis 5, dadurch gekennzeichnet, daß man als Komponente d) gegebenenfalls Etherbrücken aufweisende oder eingebaute Isocyanuratringe aufweisende (cyclo)aliphatische, mehrwertige Alkohole des Molekulargewichtsbereichs 62 -350 verwendet.

7. Verwendung der Lackpolyisocyanate gemäß Anspruch 1 in Kombination mit organischen Polyhydroxylverbindungen als Einbrennlack oder zur Herstellung eines Einbrennlacks für beliebige hitzeresistente Substrate.

8. Verwendung gemäß Anspruch 7 als Einbrennlack zur Drahtlackierung oder zur Herstellung eines Einbrennlacks zur Drahtlackierung.